Europäisches Patentamt

(19) European Patent Office

Office européen des brevets

(11) Publication number: **0 176 865**
**B1**

## EUROPEAN PATENT SPECIFICATION

(12)

(45) Date of publication of patent specification: **27.12.90**

(21) Application number: **85111770.5**

(22) Date of filing: **17.09.85**

(51) Int. Cl.⁵: **A 61 M 25/00**

(54) Steerable soft-tip catheter and method of using same.

(30) Priority: **18.09.84 US 651806**
**10.09.85 US 774345**

(43) Date of publication of application:
**09.04.86 Bulletin 86/15**

(45) Publication of the grant of the patent:
**27.12.90 Bulletin 90/52**

(84) Designated Contracting States:
**BE CH DE FR GB LI NL**

(56) References cited:
**WO-A-83/01893**
**AT-B- 361 108**
**GB-A-1 116 317**
**US-A-2 688 329**
**US-A-3 058 473**
**US-A-3 470 876**
**US-A-3 605 725**
**US-A-4 150 676**
**US-A-4 195 637**

(73) Proprietor: **Medtronic Versaflex, Inc.**
**4060B Sorrento Valley Boulevard**
**San Diego, California 92121 (US)**

(72) Inventor: **Buchbinder, Maurice**
**2323 Sharon Road**
**Menlo Park California 94025 (US)**

(74) Representative: **von der Bey, Gerhard, Dr.**
**Auenstrasse 31a**
**D-8012 Ottobrunn (DE)**

Courier Press, Leamington Spa, England.

## Description

### Field of the invention

This invention relates to steerable catheters. More particularly, this invention relates to a steerable soft-tip catheter means as stated in the precharacterising portion of claim 1. Such a catheter means is e.g. disclosed in US—A—2,688,329.

### Background of the invention

Catheters comprise tube-like members that are inserted into the body for various medical reasons, some diagnostic and others therapeutic. While in many instances the steerability or directionality of such catheters is of concern, steerability is particularly important with regard to certain urological or cardiovascular applications.

There have been various attempts to develop steerable catheters. For example, US—A—1,060,665 describes an early attempt to provide a catheter capable of some direction. However, the device disclosed in this patent, as well as catheters and catheter guides disclosed in later patents, such as US—A—2,574,840 and US—A—2,688,329, tend to be characterized by only limited directionality.

The steerable catheter means, disclosed in US—A—2 688 329 comprises a deflection wire, the distal end of which is secured to a metallic tip member protruding from the catheter. At the proximal end the deflection wire is fastened to a rod, which is axially movable by actuation of a gripping means. By actuating this gripping means the deflection wire causes deflection of the distal end portion of the catheter. For maintaining a selected deflected position of the distal end of the catheter, a nut is brought to engagement with a flange. Good steerability resp. directionality and tip control are features extremely important in the field of catheters used for cardiovascular procedures etc. In this respect the metallic tip member of the above catheter is disadvantageous, since it in a certain degree limits directionality and may restrict use in delicate applications.

In addition, some supposedly steerable catheters are too large and rigid to be of practical use in cardiovascular techniques. See, for example, US—A—3,470,876 and US—A—3,605,725, where wires equidistantly positioned along the length of a catheter are connected to a steering means which pulls on the wires to cause the distal end of the catheter to go in a desired direction. Moreover, US—A—3,521,620, US—A—3,547,103, US—A—3,625,200, and US—A—4,020,829 describe coil spring guide wires that have a certain degree of directionality but are too rigid for safe usage in certain delicate cardiovascular procedures.

According to US—A—4,033,331, a coronary catheter has a main lumen and a shaping wire lumen, when the wire is withdrawn through the shaping wire lumen, the catheter assumes certain predetermined configurations. While this so-called steerable catheter is useful in some cardiovascular applications, such as positioning the initial guiding catheter through which other devices are guided, its limited directionality and limited tip control preclude extensive use.

A medical procedure known as percutaneous transluminal coronary angioplasty (PTCA) was developed in approximately 1976—1977 by Dr. Andreas Gruntzig. According to this procedure, blockage in a coronary artery can be reduced by positioning a balloon dilatation catheter across the blockage and then inflating the balloon, which causes the blockage to decrease. Such positioning requires that the balloon dilatation catheter be "steered" into place by manipulation at the proximal end of the catheter.

The procedure is actually somewhat complex, consisting of introducing a catheter system via the femoral or brachial artery under local anesthesis. A pre-shaped guiding catheter is positioned into the orifice of the coronary artery, and through this guiding catheter a second, dilatation catheter is advanced into the branches of the coronary artery. The dilatation catheter has an elliptically shaped balloon portion near the tip which can be inflated and deflated. After traversal of the stenotic lesion of the coronary artery, the balloon portion is inflated with fluid, which dilates the lumen of the vessel.

The PTCA procedure and equipment have become increasingly refined over the past five years. The first marketable PTCA apparatus consisted of a small catheter with a single balloon port and no central lumen, that is, a so-called "fixed wire" system, which terminated in lateral openings at the distal end thereof. This system, which is the subject of US—A—4,195,637, was designed by Dr. Gruntzig and was marketed in the United States by USCI. The fixed wire catheter system disclosed in US—A—4,195,637 comprises a balloon dilatation catheter and a low friction guide catheter consisting one tubular member fitted into a more rigid shrunk-on tubular member that is not co-extensive. The distal end of the balloon dilatation catheter has a flexible tip advantageously fabricated from a spring steel wire, which serves for the detection of even the smallest passages in the hollow space or cavity which is to be treated resp. dilated.

In 1980—1981, Dr. John Simpson, working at Stanford University, began to modify the fixed wire system and eventually developed a catheter with a free central lumen for movable guide wires. This catheter system is the subject of US—A—4,323,071, which is assigned to Advanced Catheter Systems, Inc. By use of such a movable wire system, one could more readily select the desired coronary artery and get to smaller branches since the movable guide wires are inherently smaller and more flexible than the fixed wire system. Subsequent to the development of the catheter with movable guide wires, known as the Simpson-Robert system and marketed by Advanced Cardiovascular Systems, Inc. (ACS), which is a successor to Advanced Catheter Systems, Inc., USCI has abandoned the fixed wire system and has marketed a similar device, calling it the steerable catheter, "DILACA"®.

Samson, US—A—4,516,972 issued May 14, 1985, to ACS. This patent is directed to a guide catheter having a helically wound ribbon of flexible material imbedded in the wall of the catheter to provide torsional rigidity.

There is a further catheter system in use known as the Hartzler low profile catheter system. According to this catheter system a balloon dilatation catheter has a concentrically contained guide wire extending the length of said catheter. Moreover, the distal end of the guide wire extends a short distance beyond the distal end of the balloon dilatation catheter and is affixed to the distal end of the balloon dilation catheter.

The catheter system with movable guide wires and the low profile catheter system each represent an advance but still have disadvantages such as limited steerability, which is at present dependent upon the torquability, or torque control, of the movable wire. Steerability is highly significant in a cardiovascular procedure such as PTCA, or angioplasty, because less steerability results in greater time spent in the body and more possible patient trauma. Multiple insertions of guide wires and catheters can lead to thrombosis in that coagulation may commence along a guide wire surface and be forced into the heart when a catheter is slid over the guide wire. Furthermore, there are some blockages which simply can't be reached with presently known equipment.

There is definitely a need for more steerable catheter means, especially means useful in a procedure such as PTCA. Preferably such catheter means should have the following characteristics:

1. The distal end should have a pre-formed tip softer than the catheter shaft. This will allow preferential bending at the tip and thus will provide better tip control regardless of whether a hydraulic, electric, or pull wire system is used for tip control.

2. The catheter means may have an outer catheter shaft and an inner catheter to prevent bending of the inner catheter shaft, thus allowing more precise tip control.

3. The entire catheter (outer and inner) must be small enough to compare favorable with the already existing small dilatation catheters.

4. The movable steering tip curve must be precise enough to provide as close to 1:1 torque as possible. This would make the device very useful since it could ultimately be substituted for high torque wires already available.

5. The inner catheter must be free enough to rotate inside the outer catheter so that the tip may turn freely in case another turning axis is needed (superior/inferior vs. lateral).

6. The steering catheter means should optionally have a balloon inflation port, if the catheter's o.d. is small enough to be competitive with the standard dilatation catheters. If this is the case, the space between the inner and outer catheters could be used as an inflation port.

Object of the invention

The object underlying the invention is to provide a steerable catheter means having a soft tip, allowing precise steering and good tip mobility, which is in particular useful in cardiovascular applications.

This object is solved by a steerable catheter means having the features stated in claim 1. Advantageous further developments of this catheter means are indicated in the dependent claims.

Brief description of the invention

The flexible catheter tip allows easy bending of the tip away from the longitudinal axis of the catheter. This improves tip control and thereby directionality and steerability of the catheter. Furthermore, the deflection wire and catheter are rotatable about the longitudinal axis so that the catheter tip may turn freely in case another turning axis is needed.

Applicant has surprisingly developed a soft-tip, flexible and steerable cathether means, or delivery means, which is much more useful than those previously known.

Brief description of the drawings

Fig. 1 represents a planar view of an embodiment of the invention;

Fig. 2 represents a cross-sectional view along line A—A of the embodiment of the invention shown in Fig. 1;

Fig. 3 represents a partial, longitudinal, cross-sectional view of the distal end of the embodiment of the invention shown in Fig. 1;

Fig. 4 represents a planar view of the distal end of a coronary dilatation catheter as it extends from the distal end of an embodiment of the invention;

Fig. 5 depicts a patient undergoing the initial stage of a procedure in which an embodiment of the invention would be used;

Fig. 6 represents a partially sectional view where an embodiment of the invention is in a position from which a guide wire has been positioned across blockage in an artery;

Fig. 7 represents a partial longitudinal, cross-sectional view of the distal end of another embodiment of the invention;

Figs. 8, 10, 12, 14, 16, 18, 20 and 22 each represent a partial, longitudinal, cross-sectional view of a further embodiment of the invention; and

Figs. 9, 11, 13, 15, 17, 19, 21 and 23 each represent a cross-sectional view of the embodiment of the invention represented by Figs. 8, 10, 12, 14, 16, 18, 20 and 22, respectively.

Detailed description of the invention

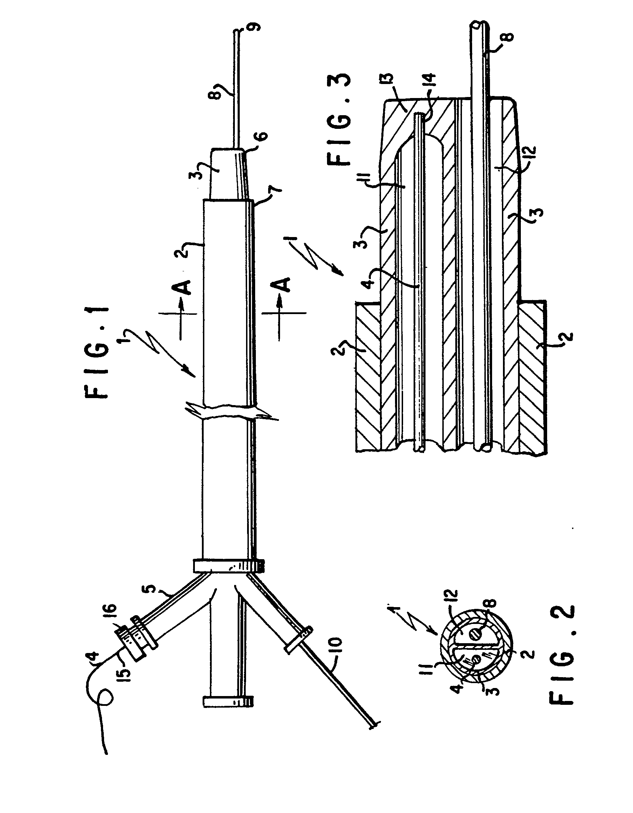

The invention can perhaps be better understood by making reference to the drawings. In Fig. 1, catheter means 1 is essentially comprised of outer catheter shaft or shell 2, inner catheter 3, steering wire 4, and control means 5. Catheter shell 2 substantially encloses inner catheter 3, which optionally is freely rotatable within catheter shaft 2.

Distal end 6 of inner catheter 3 projects out of the distal end 7 of catheter shell 2. This projection, that is, the length between distal ends 6 and 7, may vary from about 0.5 to 25 cm, preferably from about 1 to 20 cm, more preferably from about 2 to 10 cm.

A primary purpose of the invention is to provide a vehicle for delivering certain objects to parts of the body with specific steering control. In the embodiment of the invention shown in Fig. 1, a movably controlled, or movable, guide wire 8 extends the length of catheter means 1, the distal end 9 of movable guide wire 8 projecting out of inner catheter 3 and the proximal end 10 of movable guide wire 8 extending through control means 5.

As can be seen from Fig. 2 and 3, inner catheter 3 has two lumens, steering lumen 11 and open lumen 12, through which movable guide wire 8 is introduced. The distal end 13 of steering lumen 11 is closed, and the distal end 14 of steering wire 4 is embedded from about 0.1 to 7 cm, preferably from about 1 to 5 cm, into said closed distal end 13.

The proximal end 15 of steering wire 4 extends through control means 5 and is fixedly held by torque means 16. Turning torque means 16 causes wire 4 to shorten or lengthen relative to inner catheter 3, which in turn causes the distal end 6 of inner catheter 3 to bend away from the longitudinal axis of catheter shell 2.

The inner catheter 3 shown in the drawing is depicted with two lumens. However, it is within the scope of the invention that inner catheter 3 could have three or more lumens, dependent upon the particular application.

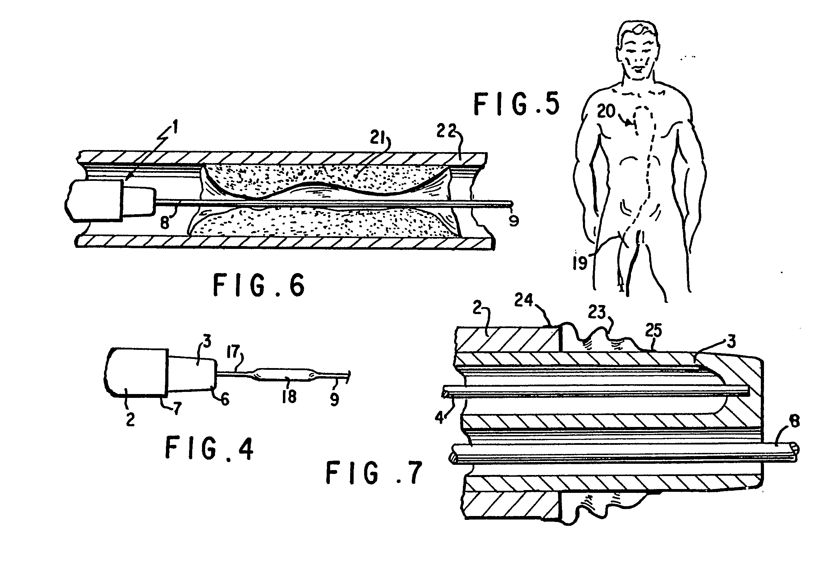

Fig. 4 shows the distal end of an embodiment of the invention with the distal portion of a balloon dilatation catheter 17 projecting beyond the distal end 6 of inner catheter 3. When ballon means 18 is inflated, the balloon means 18 presses against blockage in an artery.

The delivery system according to the invention can be, as mentioned above, used to introduce or deliver objects to various parts of the body. It is envisioned that said system will be especially useful in cardiovascular applications, such as PTCA. Use of the system in PTCA initially requires the placement of a lead cardiac catheter, such as is shown, for example, in Fig. 5. A cutaneous opening 19 through the skin into the femoral artery in the upper thigh is formed, and then the lead cardiac catheter 20 is positioned essentially as shown. Next, a steerable catheter means according to the invention would be threaded into the lead cardiac catheter until the distal end of said catheter means protrudes from the distal end of the lead cardiac catheter. Then, the catheter means according to the invention would be steered to the desired position, such as adjacent to a blockage in an artery, at which time a movable guide wire or a balloon dilatation catheter could be maneouvered across the blockage.

Such a blockage 21 in an artery 22 is shown in Fig. 6. The movable guide wire 8 has been manipulated across the blockage 21 so that distal end 9 of movable guide 8 extends past blockage 21. Once the guide wire 8 is in proper position, a balloon dilatation catheter is then threaded along movable guide wire 8 until it is also in position across blockage 21.

Another embodiment of the invention is presented by Fig. 7, where a dilatation balloon means 23 is attached to catheter shell 2 at point 24 and to inner catheter 3 at point 25. The dilatation balloon means 23 is inflated by a non-compressible fluid, preferably a physiological salt solution and/or an iodinated x-ray contrast medium, that is furnished through the space between catheter shell 2 and inner catheter 3, that is, said space acts as a balloon inflation port. The approximate longitudinal distance between points 24 and 25 is from about 2 to 20 cm, preferably from about 3 to 10 cm.

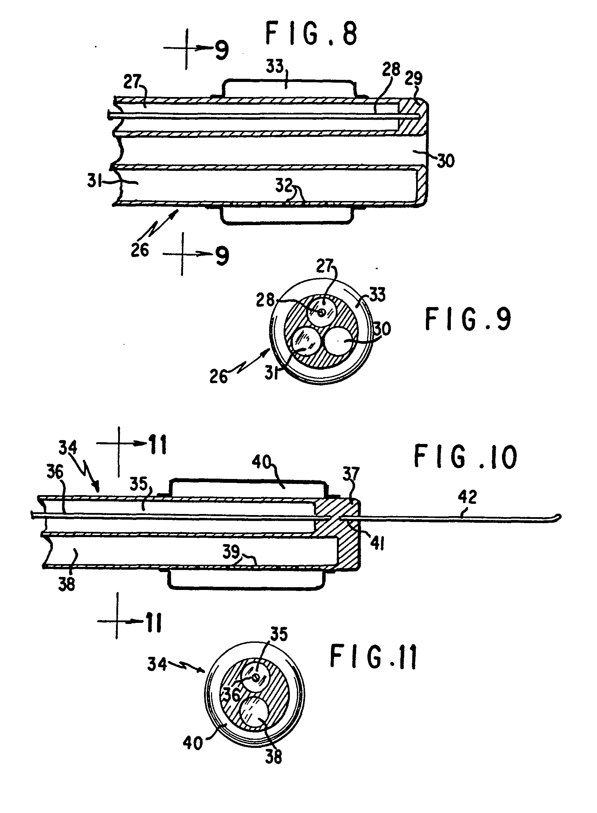

Further embodiments of the invention are set forth in Figs. 8 to 23. The embodiment represented by Figs. 8 and 9 comprises a triple lumen catheter 26 wherein steering lumen 27 has a steering wire 28 embedded in the distal end 29 of steering lumen 27. Open lumen 30 provides a passageway for, for example, a movable wire or a retractable pressure sensing fiber (not shown). Balloon dilatation lumen 31 has openings 32 near its distal end to permit inflation of dilatation balloon 33.

Figs. 10 to 13 depict double lumen catheter means according to the invention. In Fig. 10, the catheter 34 comprises steering lumen 35 having a steering wire 36 embedded in the distal end 37 of steering lumen 35. Balloon inflation lumen 38 has opening 39 near its distal end to permit inflation of dilatation balloon 40. Also embedded in distal end 37 is the proximal end 41 of a floppy wire flexible tip, or "antenna", 42, which acts primarily to protect the inner surfaces as the catheter is maneouvred through various body passageways and/or to detect small passages in the hollow space or cavity to be dilated. Advantageously the antenna is fabricated from a spring steel wire and is from about 0.5 to 5 cm, preferably from about 1 to 3 cm, in length.

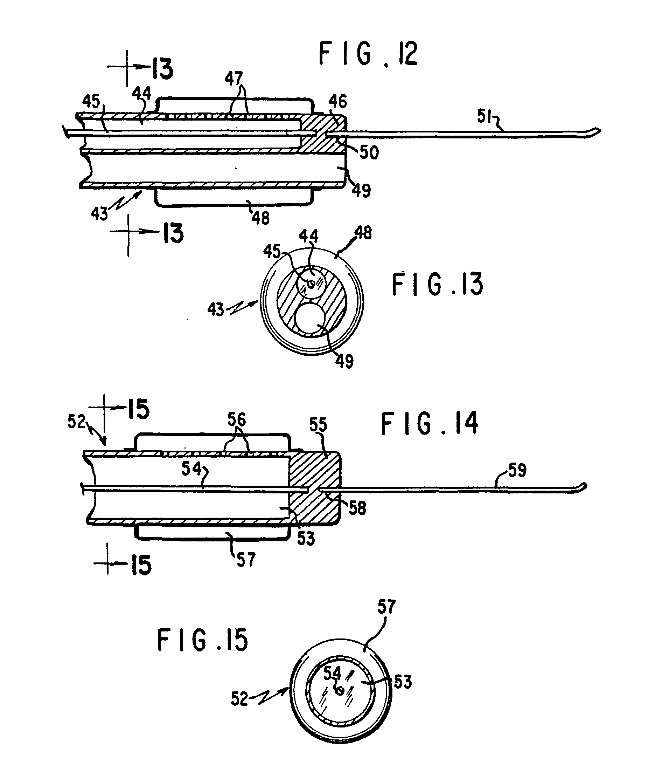

Double lumen catheter 43 represented in Figs. 12 and 13 has a steering lumen 44 having a steering wire 45 embedded in its distal end 46, said lumen 44 also having openings 47 near distal end 46 to permit inflation of dilatation balloon 48. Open lumen 49 provides a conduit for an object such as a retractable pressure sensing fiber (not shown). The proximal end 50 of an antenna 51 is affixed to the distal end of catheter 43.

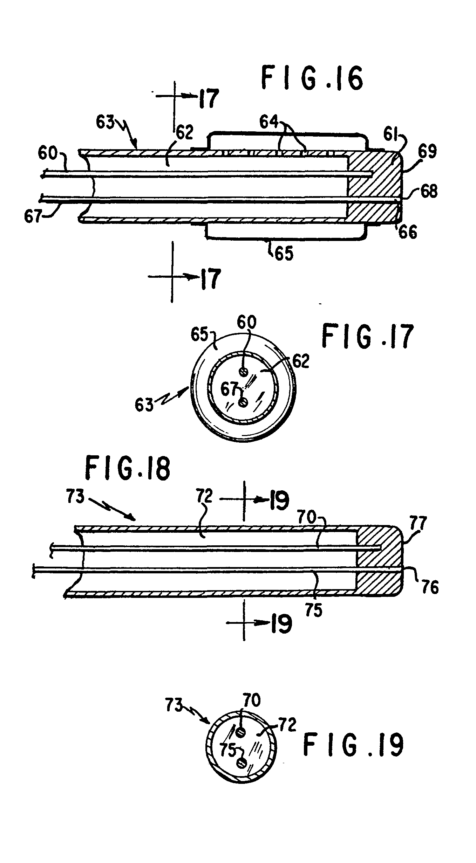

The embodiments of the invention shown in Figs. 14 and 19 are single lumen catheter means. The catheter 52 in Figs. 14 and 15 comprises a closed lumen 53 having a steering wire 54 embedded in the distal end 55 of closed lumen 53. Closed lumen 53 has openings 56 near distal end 55 to permit inflation of dilatation balloon 57. The proximal end 58 of antenna 59 is also embedded in distal end 55.

According to Figs. 16 and 17, steering wire 60 is embedded in or near distal end 61 of closed lumen 62 of catheter 63. Lumen 62 has openings 64 near distal end 61 to permit inflation of dilatation balloon 65. Also, the distal end 66 of a pressure sensing fiber 67 is embedded in distal end 61, the outer surface 68 of pressure sensing fiber distal end 66 being substantially flush with the outer surface 69 of lumen distal end 61. The pressure sensing fiber 67 is a very small fiber which transduces the pressure sensed at distal end 66. Fiber 67 is advantageously from about 1 to 10 microns, preferably from about 5 to 8 microns, in diameter.

The embodiment of the invention set forth in Figs. 18 and 19 is similar to the embodiment set forth in Figs. 16 and 17. Steering wire 70 is embedded in the distal end 71 of lumen 72 of catheter 73. The distal end 74 of a pressure sensing fiber 75 is embedded in distal end 71, the outer surface 76 of pressure sensing fiber distal end 72 being substantially flush with the outer surface 77 of lumen distal end 71.

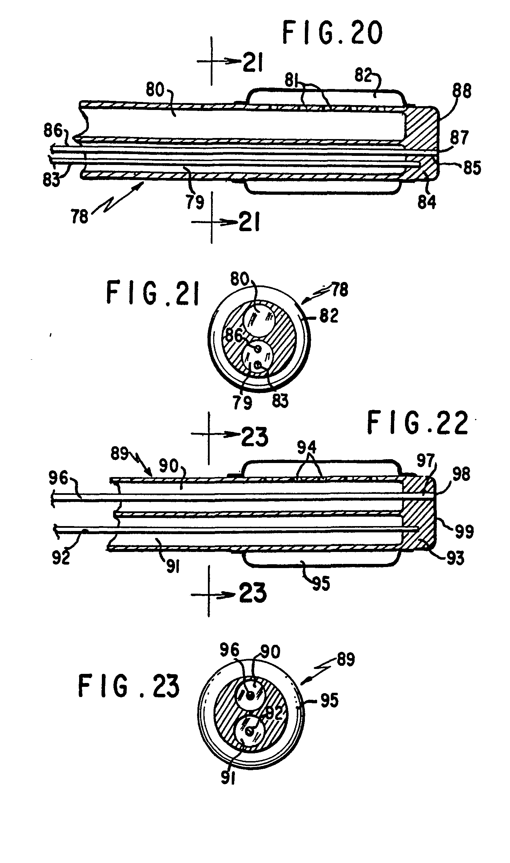

Fig. 20 to 23 represent double lumen embodiments having a pressure sensing fiber and a steering wire. According to Figs. 20 and 21, catheter 78 comprises steering lumen 79 and balloon inflation lumen 80, which has openings 81 near its distal end to permit inflation of dilatation balloon 82. Steering lumen 79 has a steering wire 83 of a pressure sensing fiber 86 is also embedded in distal end 84, the outer surface 87 of pressure sensing fiber distal end 85 being substantially flush with the outer surface 88 of the distal end of catheter 78.

In Figs. 22 and 23, catheter 89 comprises balloon inflation lumen 90 and steering lumen 91, wherein steering wire 92 is embedded in the distal end 93 of steering lumen 91. Balloon inflation lumen 90 has (1) openings 94 to permit inflation of dilatation balloon 95 and (2) a pressure sensing fiber 96. The distal end 97 of pressure sensing fiber 96 is embedded in the distal end of balloon inflation lumen 90 in a manner such that the outer surface 98 of pressure sensing fiber distal end 97 is substantially flush with the outer surface 99 of the distal end of catheter 89.

In the embodiments of the invention described in Figs. 8 to 23, the distal ends of the respective catheter means are manoeuvered across a blockage in an artery. More particularly, the dilatation balloon means are positioned so that they can be inflated to cause the blockage to decrease. Also, the pressure sensing fibers can make desired pressure readings.

Each of the embodiments of the invention set forth in Figs. 12 to 23 is attached to a suitable control means, having torque control, such as that shown in Fig. 1. It is important to note that according to the invention the control means has both push-pull and rotational motion/capability to properly direct the catheter.

Reference has been made to openings 32, 39, 47, 56, 64, 81 and 94, which permit inflation of dilatation balloon means. Said openings should be of sufficient size and in sufficient number to permit quick inflation but not to weaken the catheter means structurally. For example, said openings could comprise from 2 to 12 substantially circular or rectangular openings each having a cross-sectional area of from about .01 to 25 mm$^2$, preferably from about .05 to 15 mm$^2$.

The inflation and deflation of dilatation balloon means are well-known techniques, and various equipment therefor has been developed. See, for example, US—A—4,231,715, US—A—4,332,254 and US—A—4,439,185, and US—S—274,470.

The particular dimensions of the various aspects of the steerable means according to the invention would be readily apparent to those skilled in the art. The outer catheter shell should be from about 90 to 150 cm, preferably from about 100 to 150 cm, more preferably from about 125 to 145 cm, in length, and the movable inner catheter should be about 1 to 20 cm longer, preferably from about 2 to 10 cm longer. For example, the outer catheter and inner catheter could be about 135 cm and 140 cm in length, respectively. Where there is only a single catheter, said catheter is from about 90 to 170 cm, preferably from about 125 to 165 cm, in length. Guide wires can be from about 150 to 400 cm, preferably about 225 to 325 cm, in length.

The respective diameters of the catheters and lumens will vary according to application. The o.d. of the outer catheter or a single catheter could be from about 0.010 to 0.50 inches, preferably from about 0.030 to 0.30 inches, with or without balloon ports. The inner diameters of each of the lumens in the inner catheter or a single catheter could each be from about 0.0003 to 0.30 inches, preferably from about 0.005 to 0.200 inches, more preferably from about 0.018 to 0.050 inches. Also, there may be a space of from about 0.001 to 0.10 inches, preferably from about 0.005 to 0.025 inches, between the outer surface of the inner catheter and the inner surface of the outer catheter, which space could be used for inflation.

The catheter means of this invention may be formed from any suitable plastic material having a low coefficient of friction, such as plasticized vinyl resins, a polyolefin such as polyethylene, polyvinylchloride, polyamide, synthetic and natural rubbers, and polyurethane elastomers. In most instances the catheter means will have one or more substances or elements to make it radiopaque, so that it can be readily seen by a fluoroscope during medical and/or surgical procedurs. Various marking elements which are impervious or only slightly pervious to radiation, such as gold foil, are known for this purpose. The various guide wires useful according to the invention may be any suitable surgical metal or other material which is medically non-reactive.

Although the discussion above relates to use of a balloon dilatation catheter, the invention is not limited to this concept. The catheter means of the invention is essentially a delivery system capable of delivering various objects to desired parts of the body, and the delivery of fluids such as radiopaque fluids, pharmaceuticals, or of other medical devices such as, for example, optical fibers such as quartz fibers useful in delivering laser energy, is within the scope of the invention.

The preceding specific embodiments are illustrative of the practice of the invention. It is to be understood, however, that other expedients known to those skilled in the art or disclosed herein, may be employed without departing from the scope of the appended claims.

**Claims**

1. A steerable catheter means, comprising:
a flexible catheter (3) having distal (6) and proximal ends and one or more lumens (11, 12; 27, 30, 31; 35, 38; 44, 49; 53; 62, 72; 79, 80; 90, 91) extending therethrough, at least one (11; 27, 31; 35, 38; 44; 53; 62; 72; 79, 80; 90, 91) of the lumens being closed at its distal end;
a deflection wire (4; 28; 36; 45; 54; 60; 70; 83; 92) having distal and proximal ends and axially extending through a lumen having a closed end (11; 27; 35; 44; 53; 62; 72; 79; 91), the distal end (14) of the deflection wire being fixedly embedded in the closed end (13; 29; 37; 46; 55; 61; 93),
control means (5) attached to the proximal end of the catheter, the proximal end of the deflection wire extending through the control means and the control means having engaging means (16) which fixedly engages the deflection wire for displacement distally and proximally; characterised in that
the flexible catheter (3) has a flexible tip softer than the catheter shaft and
the engaging means (16) of the control means (5) is a torque means, by turning of which the length of deflection wire (4; 28; 36; 45; 54; 60; 70; 83; 92) is increased or decreased relative to the catheter length, to thereby bend the catheter tip.

2. Catheter means as claimed in claim 1, characterised in that the engaging means (16) is provided for push-pull and rotational engagement.

3. Catheter means as claimed in claim 1 or 2, characterised in that the catheter comprises from 1 to 3 lumens.

4. Catheter means as claimed in any of claims 1 to 3, characterised in that the catheter has at least two lumens and one (12; 30; 49) of the lumens is open.

5. Catheter means as claimed in claim 4, characterised in that the open lumen (30) is capable of having a balloon dilatation catheter (17) or a retractable pressure sensor threaded therethrough.

6. Catheter means as claimed in any of claims 1 to 5, characterised in that the outer surface of the flexible catheter (3) and/or of a balloon dilatation catheter (17) near the distal portion thereof comprises dilatation balloon means (23; 33; 40; 48; 57; 65; 82; 95), the distal end of the balloon dilatation catheter extending through a passageway in the control means (5).

7. Catheter means as claimed in claim 6, characterised in that a closed lumen (31; 38; 44; 53; 62; 80; 90) has openings (32; 39; 47; 57; 64; 81; 94) near its distal end and the openings are arranged to permit inflation of the dilatation balloon means (33; 40; 48; 56; 65; 82; 95).

8. Catheter means as claimed in any of claims 5 to 7, characterised in that the closed lumen (62) has a pressure sensing fiber (67; 75; 86) extending therein, the pressure sensing fiber having distal and proximal ends and the distal end of the pressure sensing fiber extending through the distal end (61; 71; 84) of the catheter means to the outer surface (69; 77; 87) thereof.

9. Catheter means as claimed in claim 8, characterised in that the distal end of the pressure sensing fiber (67; 75; 86) is substantially flush with the outer surface (69; 77; 87) of the distal end (61; 71; 84) of the catheter means.

10. Catheter means as claimed in any of claims 1 to 9, characterised in that the flexible tip (42; 51; 59) has proximal (41; 50; 58) and distal (37; 46; 55) ends, the proximal end being affixed or embedded in the distal end of the catheter means.

11. Catheter means as claimed in claim 10, characterised in that the flexible tip (42; 51; 59) is a floppy wire comprised of spring steel wire.

12. Catheter means as claimed in any of claims 3 to 11, characterised in that the catheter means comprises a lumen open at its distal end and the proximal end of the lumen is in fluid communication with a passageway in the control means.

13. Cathether means as claimed in any of claims 1 to 12, characterised in that the flexible catheter (3) extends through an outer catheter member (2) having distal and proximal ends and the distal end of the flexible catheter protrudes distally beyond the distal end of the outer catheter member.

14. Catheter means as claimed in claim 13, the flexible catheter having at least one lumen being open distally, characterised in that the control means (5) has a passageway in fluid connection with at least one open lumen.

15. Catheter means as claimed in claim 14, characterised in that it comprises a guide wire (8) extending through an open lumen (12), the guide wire having distal and proximal ends, the distal end (9) of the guide wire protruding distally beyond the distal end of the flexible catheter, and the proximal end (10) of the guide wire extending through the passageway in the control means (5).

16. Catheter means as claimed in any of claims 4 to 15, having a balloon dilatation catheter, characterised in that the balloon dilatation catheter has a central lumen therein and a guide wire having proximal and distal ends extends the length of the central lumen, the distal end of the balloon dilatation catheter protruding distally beyond the distal end of the flexible catheter and the distal end of the guide wire extending distally beyond the distal end of the balloon dilatation catheter.

17. Catheter means as claimed in any of claims 13 to 16, characterised in that the flexible catheter (3) is freely movable within the outer catheter member (2).

18. Catheter means as claimed in any of claims 1 to 17, characterised in that the deflection wire is tapered at its distal end.

**Patentansprüche**

1. Lenkbare Kathetereinrichtung, aufweisend
einen flexiblen Katheter (3) mit distalen (6) und proximalen Enden und einem oder mehreren sich hindurch erstreckenden Lumina (11, 12; 27, 30, 31; 35, 38; 44, 49; 53; 62; 72; 79, 80; 90, 91), wobei wenigstens eines (11; 27, 31; 35, 38; 44; 53; 62; 72; 79, 80; 90, 91) der Lumina am distalen Ende geschlossen ist;
einen Ablenkdraht (4; 28; 36; 45; 54; 60; 70; 83; 92), der distale und proximale Enden aufweist und sich axial durch ein Lumen mit einem geschlossen Ende (11; 27; 35; 44; 53; 62; 72; 79; 91) erstreckt, wobei das distale Ende (14) des Ablenkdrahtes fest im geschlossenen Ende (13; 29; 37; 46; 55; 61; 93) eingebettet ist,
eine am proximalen Ende des Katheters angebrachte Bedieneinrichtung (5), wobei sich das proximale Ende des Ablenkdrahtes durch die Bedieneinrichtung erstreckt und die Bedieneinrichtung eine Eingriffseinrichtung (16) aufweist, die fest mit dem Ablenkdraht zur distalen und proximalen Verstellung in Eingriff steht; dadurch gekennzeichnet, daß
der flexible Katheter (3) eine flexible Spitze aufweist, die weicher als der Katheterschaft ist, und
die Eingriffseinrichtung (16) der Bedieneinrichtung (5) eine Verdreheinrichtung ist, durch deren Drehen die Länge des Ablenkdrahtes (4; 28; 36; 45; 54; 60; 70; 83; 92) in bezug auf die Katheterlänge vergrößert oder verringert wird, um auf diese Weise die Katheterspitze zu biegen.

2. Kathetereinrichtung nach Anspruch 1, dadurch gekennzeichnet, daß die Eingriffseinrichtung (16) für einen Schub-Zug- und Dreheingriff vorgesehen ist.

3. Kathetereinrichtung nach Anspruch 1 oder 2, dadurch gekennzeichnet, daß der Katheter ein bis drei Lumina aufweist.

4. Kathetereinrichtung nach einem beliebigen der Ansprüche 1 bis 3, dadurch gekennzeichnet, daß der Katheter wenigstens zwei Lumina aufweist und eines (12; 30; 49) der Lumina offen ist.

5. Kathetereinrichtung nach Anspruch 4, dadurch gekennzeichnet, daß das offene Lumen (30) in der Lage ist, einen Ballondilatationskatheter (17) oder einen durchgeführten, zurückziehbaren Drucksensor aufzuweisen.

6. Kathetereinrichtung nach einem beliebigen der Ansprüche 1 bis 5, dadurch gekennzeichnet, daß die Außenseite des flexiblen Katheters (3) und/oder eines Ballondilatationskatheters (17) nahe seines distalen Abschnittes eine Dilatationsballoneinrichtung (23; 33; 40; 48; 57; 65; 82; 95) aufweist, wobei sich das distale Ende des Ballondilatationskatheters durch eine Durchführung in der Bedieneinrichtung (5) erstreckt.

7. Kathetereinrichtung nach Anspruch 6, dadurch gekennzeichnet, daß ein geschlossenes Lumen (31; 38; 44; 53; 62; 80; 90) Öffnungen (32; 39; 47; 57; 64; 81; 94) in der Nähe des distalen Endes aufweist und die Öffnungen so angeordnet sind, daß sie das Füllen der Dilatationsballoneinrichtung (33; 40; 48; 56; 65; 82; 95) gestatten.

8. Kathetereinrichtung nach einem beliebigen der Ansprüche 5 bis 7, dadurch gekennzeichnet, daß das geschlossene Lumen (62) eine sich hindurch erstreckende Druckabfühlfaser (67; 75; 86) aufweist, wobei die Druckabfühlfaser distale und proximale Ende aufweist und sich das distale Ende der Druckabfühlfaser durch das distale Ende (61; 71; 84) der Kathetereinrichtung zu deren Aussenseite (69; 77; 87) hin erstreckt.

9. Kathetereinrichtung nach Anspruch 8, dadurch gekennzeichnet, daß das distale Ende der Druckabfühlfaser (67; 75; 86) im wesentlich fluchtend mit der Außenseite (69; 77; 87) des distalen Endes (61; 71; 84) der Kathetereinrichtung ist.

10. Kathetereinrichtung nach einem beliebigen der Ansprüche 1 bis 9, dadurch gekennzeichnet, daß die flexible Spitze (42; 51; 59) proximale (41; 50; 58) und distale (37; 46; 55) Enden aufweist, wobei das proximale Ende im distalen Ende der Kathetereinrichtung befestigt oder eingebettet ist.

11. Kathetereinrichtung nach Anspruch 10, dadurch gekennzeichnet, daß die flexible Spitze (42; 51; 59) ein aus einem Federstahldraht bestehender flexibler Draht ist.

12. Kathetereinrichtung nach einem beliebigen der Ansprüche 3 bis 11, dadurch gekennzeichnet, daß die Kathetereinrichtung ein am distalen Ende offenes Lumen aufweist und sich das proximale Ende des Lumens in Fluidverbindung mit einer Durchführung in der Bedieneinrichtung befindet.

13. Kathetereinrichtung nach einem der Ansprüche 1 bis 12, dadurch gekennzeichnet, daß sich der flexible Katheter (3) durch ein distale und proximale Enden aufweisendes Außenkatheterelement (2) erstreckt und das distale Ende des flexiblen Katheters in distaler Richtung über das distale Ende des Außenkatheterelementes vorsteht.

14. Kathetereinrichtung nach Anspruch 13, bei der der flexible Katheter wenigstens ein in distaler Richtung offenes Lumen aufweist, dadurch gekennzeichnet, daß die Bedieneinrichtung (5) eine sich in Fluidverbindung mit wenigstens einem offenen Lumen befindende Durchführung aufweist.

15. Kathetereinrichtung nach Anspruch 14, dadurch gekennzeichnet, daß sie einen sich durch ein offenes Lumen (12) erstreckenden Führungsdraht (8) aufweist, wobei der Führungsdraht distale und proximale Enden aufweist, wobei das distale Ende (9) des Führungsdrahtes in distaler Richtung über das distale Ende des flexiblen Katheters hinaus vorsteht und sich das proximale Ende (10) des Führungsdrahtes durch die Durchführung in der Bedieneinrichtung (5) erstreckt.

16. Kathetereinrichtung nach einem beliebigen der Ansprüche 4 bis 15, aufweisend einen Ballondilatationskatheter, dadurch gekennzeichnet, daß der Ballondilatationskatheter ein zentrales Lumen aufweist und sich ein proximale und distale Enden aufweisender Führungsdraht längs des zentralen Lumens erstreckt, wobei das distale Ende des Ballondilatationskatheters in distaler Richtung über das distale Ende des flexiblen Katheters vorsteht und sich das distale Ende des Führungsdrahtes in distaler Richtung über das distale Ende des Ballondilatationskatheters hinaus erstreckt.

17. Kathetereinrichtung nach einem beliebigen der Ansprüche 13 bis 16, dadurch gekennzeichnet, daß der flexible Katheter (3) innerhalb des Außenkatheterelementes (2) frei beweglich ist.

18. Kathetereinrichtung nach einem der Ansprüche 1 bis 17, dadurch gekennzeichnet, daß sich der Ablenkdraht (3) am distalen Ende verjüngt.

**Revendications**

1. Dispositif orientable à cathéter, comprenant un cathéter souple (3) ayant des extrémités distale (6) et proximale et une ou plusieurs lumières (11, 12; 27, 30, 31; 35, 38; 44, 49; 53; 62; 72; 79, 80; 90, 91) qui y sont formées, l'une au moins des lumières (11; 27, 31; 35, 38; 44; 53; 62; 72; 79, 80; 90, 91) étant fermée à son extrémité distale,
un fil de déviation (4; 28; 36; 45; 54; 60; 70; 83; 92) ayant des extrémités distale et proximale est disposé axialement dans une lumière ayant une extrémité fermée (11; 27; 35; 44; 53; 62; 72; 79; 91), l'extrémité distale (14) du fil de déviation étant enrobée à demeure dans l'extrémité fermée (13; 29; 37; 46; 55; 61; 93), et
un dispositif de commande (5) fixé à l'extrémité proximale du cathéter, l'extrémité proximale du fil de déviation passant dans le dispositif de commande et le dispositif de commande comprenant un dispositif de coopération (16) qui coopère à demeure avec le fil de déviation afin qu'il le déplace vers l'extrémité distale et vers l'extrémité proximale, caractérisé en ce que
le cathéter souple (3) a une pointe plus souple que la tige du cathéter, et
le dispositif de coopération (16) du dispositif de commande (5) est un dispositif d'application de couple grâce à la rotation duquel la longueur du fil de déviation (4; 28; 36; 45; 54; 60; 70; 83; 92) est augmentée ou réduite par rapport à la longueur du cathéter de manière que la pointe du cathéter fléchisse.

2. Dispositif à cathéter selon la revendication 1, caractérisé en ce que le dispositif de coopération (16) est destiné à coopérer par poussée-traction et par rotation.

3. Dispositif à cathéter selon la revendication 1 ou 2, caractérisé en ce que le cathéter comporte une à trois lumières.

4. Dispositif à cathéter selon l'une quelconque des revendications 1 à 3, caractérisé en ce que le cathéter a au moins deux lumières et l'une des lumières (12; 30; 49) est ouverte.

5. Dispositif à cathéter selon la revendication 4, caractérisé en ce que la lumière ouverte (30) peut avoir un cathéter (17) de dilatation de ballon ou un capteur rétractable de pression enfilé à l'intérieur.

6. Dispositif à cathéter selon l'une quelconque des revendications 1 à 5, caractérisé en ce que la surface externe du cathéter souple (3) et/ou du cathéter (17) de dilatation de ballon, près de son extrémité distale, comporte un dispositif à ballon de dilatation (23; 33; 40; 48; 57; 65; 82; 95), l'extrémité distale du cathéter de dilatation de ballon passant dans un passage formé dans le dispositif de commande (5).

7. Dispositif à cathéter selon la revendication 6, caractérisé en ce qu'une lumière fermée (31; 38; 44; 53; 62; 80; 90) a des ouvertures (32; 39; 47; 57; 64; 81; 94) prés de son extrémité distale et les ouvertures sont destinées à permettre le gonflage du dispositif à ballon de dilatation (33; 40; 48; 56; 65; 82; 95).

8. Dispositif à cathéter selon l'une quelconque des revendications 5 à 7, caractérisé en ce que la lumière fermée (62) a une fibre (67; 75; 86) de détection de pression qui est placée dans cette lumière, la fibre de détection de pression ayant des extrémités distale et proximale, et l'extrémité distale de la fibre de détection de pression est disposée dans l'extrémité distale (61; 71; 84) du dispositif à cathéter vers la surface externe (69; 77; 87) de celui-ci.

9. Dispositif à cathéter selon la revendication 8, caractérisé en ce que l'extrémité distale de la fibre de détection de pression (67; 75; 86) est pratiquement au niveau de la surface externe (69; 77; 87) de l'extrémité distale (61; 71; 84) du dispositif à cathéter.

10. Dispositif à cathéter selon l'une quelconque des revendications 1 à 9, caractérisé en ce que la pointe souple (42; 51; 59) a des extrémités proximale (41; 50; 58) et distale (37; 46; 55), l'extrémité proximale étant fixée ou enrobée à l'extrémité distale du dispositif à cathéter.

11. Dispositif à cathéter selon la revendication 10, caractérisé en ce que la pointe souple (42; 51; 59) est un fil souple constitué d'un fil d'acier à ressort.

12. Dispositif à cathéter selon l'une quelconque des revendications 3 à 11, caractérisé en ce que le dispositif à cathéter comporte une lumière ouverte à son extrémité distale, l'extrémité proximale de la lumière étant en communication avec un passage formé dans le dispositif de commande pour la circulation d'un fluide.

13. Dispositif à cathéter selon l'une quelconque des revendications 1 à 12, caractérisé en ce que le cathéter souple (3) passe dans un organe externe (2) de cathéter ayant des extrémités distale et proximale, et l'extrémité distale du cathéter souple dépasse vers l'extérieur au-delà de l'extrémité distale de l'organe externe de cathéter.

14. Dispositif à cathéter selon la revendication 13, du type dans lequel le cathéter souple a au moins une lumière ouverte à l'extrémité distale, caractérisé en ce que le dispositif de commande (5) a un passage communiquant avec au moins une lumière ouverte afin qu'un fluide puisse circuler.

15. Dispositif à cathéter selon la revendication 14, caractérisé en ce qu'il comporte un fil de guidage (8) passant dans une lumière ouverte (12), le fil de guidage ayant des extrémités distale et proximale, l'extrémité distale (9) du fil de guidage dépassant à l'extrémité distale au-delà de l'extrémité distale du cathéter souple, et l'extrémité proximale (10) du fil de guidage passant dans le passage formé dans le dispositif de commande (5).

16. Dispositif à cathéter selon l'une quelconque des revendications 4 à 15, ayant un cathéter de dilatation de ballon, caractérisé en ce que le cathéter de dilatation de ballon a une lumière centrale et un fil de guidage ayant des extrémités proximale et distale et disposé sur toute la longueur de la lumière centrale, l'extrémité distale du cathéter de dilatation de ballon dépassant au-delà de l'extrémité distale du cathéter souple et l'extrémité distale du fil de guidage dépassant au-delà de l'extrémité distale du cathéter de dilatation de ballon.

17. Dispositif à cathéter selon l'une quelconque des revendications 13 à 16, caractérisé en ce que le cathéter souple (3) est mobile librement dans l'organe externe (2) de cathéter.

18. Dispositif à cathéter selon l'une quelconque des revendications 1 à 17, caractérisé en ce que le fil de déviation a une dimension qui diminue progressivement à son extrémité distale.

FIG.1

FIG.2

FIG.3

EP 0 176 865 B1

FIG.5

FIG.6

FIG.7

FIG.4

FIG.8

FIG.9

FIG.10

FIG.11

3

FIG.12

FIG.13

FIG.14

FIG.15

FIG.16

FIG.17

FIG.18

FIG.19

FIG.20

FIG.21

FIG.22

FIG.23